(19) Europäisches Patentamt
European Patent Office
Office européen des brevets

(11) Publication number : 0 325 375 B1

# (12) EUROPEAN PATENT SPECIFICATION

(45) Date of publication of patent specification : 12.08.92 Bulletin 92/33

(51) Int. Cl.[5] : **C07D 403/06**, C07D 231/54, C07D 401/06, C07D 413/06, C07D 417/06, A61K 31/415, A61K 31/42, A61K 31/425

(21) Application number : 89300293.1

(22) Date of filing : 13.01.89

## (54) 1H-indazole-3-acetic acids as aldose reductase inhibitors.

The file contains technical information submitted after the application was filed and not included in this specification

(30) Priority : 19.01.88 PCT/US88/00110

(43) Date of publication of application : 26.07.89 Bulletin 89/30

(45) Publication of the grant of the patent : 12.08.92 Bulletin 92/33

(84) Designated Contracting States : AT BE CH DE ES FR GB GR IT LI LU NL SE

(56) References cited :
EP-A- 0 222 576
EP-A- 0 295 051
CHEMICAL ABSTRACTS, vol. 83, no. 1, July 7, 1975, Columbus, Ohio, USA; WADA H. et al.: "Indazole-3-acetic acid derivativesand salts", page 847, column 1, abstract-no. 10 069g

(56) References cited :
CHEMICAL ABSTRACTS, vol. 100, no. 7, February 13, 1984, Columbus, Ohio, USA; FUJISAWA PHARMACEUTICAL CO. Ltd.:"Indazoleacetic acid derivatives", page 580, column 2, abstract-no. 51 567f
CHEMICAL ABSTRACTS, vol. 85, no. 1, July 5, 1976, Columbus, Ohio, USA; CORSI G. et al.:"1-halobenzyl-1H-indazole-3-carboxylic acids. A new class of antispermatogenic agents", page 11, column 1, abstract-no. 179v

(73) Proprietor : PFIZER INC.
235 East 42nd Street
New York, N.Y. 10017 (US)

(72) Inventor : Mylari, Banavara Lakshman
6, Quinley Way
Waterford Connecticut (US)
Inventor : Zembrowski, William James
1315 Route 163
Oakdale Connecticut (US)

(74) Representative : Moore, James William, Dr.
Pfizer Limited Ramsgate Road
Sandwich Kent CT13 9NJ (GB)

EP 0 325 375 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention is concerned with 1H-indazole-3-acetic acid derivatives and their pharmaceutically-acceptable esters and salts. By means of their inhibition of the aldose reductase enzyme, these compounds are useful in the treatment of diabetic complications.

Compounds, such as sorbinil (S-6-fluorospiro[chroman-4,4'-imidazoline]-2',5'-dione; Sarges, U.S. Patent No. 4,130,714), which have aldose reductase inhibitory activity, are of value in controlling certain chronic complications arising from diabetes mellitus (e.g., diabetic cataracts and neuropathy).

Non-hydantoin compounds previously reported to inhibit aldose reductase include 1H-benz[d,e]isoquinoline-1,3(2H)-dione-2-acetic acid derivatives, Sestanj et al., US-A-3,821,383; halogen substituted chroman-4-carboxylic and chroman-4-acetic acids, Belletire, US-A-4,210,663; spiro[chroman-4,5'-oxazolidin]-2',3'-diones, Schnur, U.S. Patent No. 4,200,642; and variously substituted phthalazin-1(2H)-on-4-acetic acids, Larson et al., EP-A No. 222,576 and EP-A-0295051.

Variously substituted 1-benzyl-1H-indazole-3-carboxylic acids and specifically 1-(p-chlorobenzyl)-1H-3-acetic acid have been reported to be useful as antispermatogenic agents, Corsi et al., J. Med. Chem., vol. 19, pp. 778-783 (1976) (C.A. 85 (1976), 179v).

C.A. 83 (1975), 10069g and C.A. 100 (1984), 51567f refer to Indazole-3-acetic acid derivatives which are useful as analgesics, antipyrethics and antiinflammatory agents and as growth inhibitors and herbicides respectively.

The present invention is directed to compounds having the formula

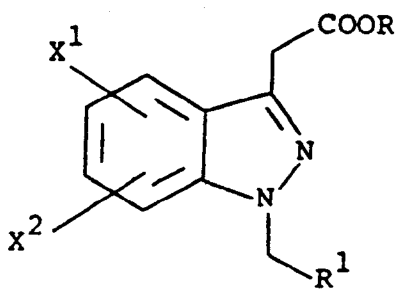

---(I)

wherein

$X^1$ and $X^2$ are each independently hydrogen, fluoro, chloro, bromo, trifluoromethyl, $(C_1-C_3)$alkyl or $(C_1-C_3)$alkoxy;

R is hydrogen or a radical group forming a conventional ester which is hydrolyzable under physiological conditions;

$R^1$ is

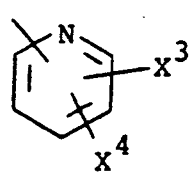 , 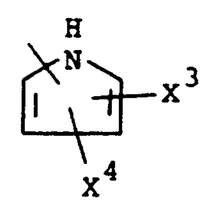 , 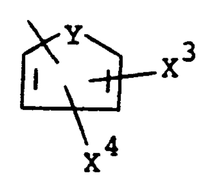 ,

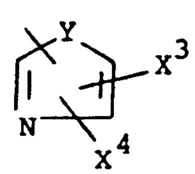 , 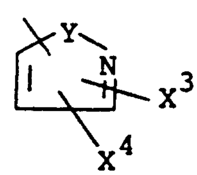 , 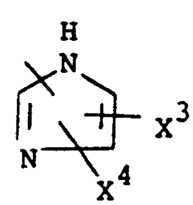 ,

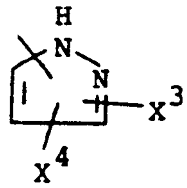 or 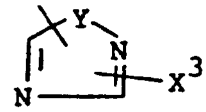 ;

Y is sulfur or oxygen;

$X^3$, when taken separately, is hydrogen, fluoro, chloro, bromo, trifluoromethyl, $(C_1\text{-}C_3)$alkyl, $(C_1\text{-}C_3)$alkoxy or

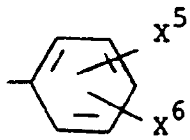 ;

$X^4$, when taken separately, $X^5$ and $X^6$ are each independently hydrogen, fluoro, chloro, bromo, trifluoromethyl, $(C_1\text{-}C_3)$alkyl or $(C_1\text{-}C_3)$alkoxy; and

$X^3$ and $X^4$, when taken together, are combined with the adjacent carbons to which they are attached to form a benzene ring substituted by $X^5$ and $X^6$;

the pharmaceutically acceptable cationic salts thereof when R is hydrogen; and

the pharmaceutically acceptable acid addition salts thereof.

Esters of acidic pharmaceutical compounds (such as penicillins and non-steroidal antiinflammatory agents) which are hydrolyzed under physiological conditions (sometimes referred to as pro-drug esters) are becoming as common as pharmaceutically acceptable salts in the pharmaceutical art. Of particular value in the present instance are those esters wherein R is:

1H-furan-5-on-1-yl;

1H-isobenzofuran-3-on-1-yl;

gamma-butyrolacton-4-yl;

$-CH_2CH_2NR^2R^3$;

$-CHR^4OCOR^5$; or

$-CHR^4OCOOR^6$;

wherein

$R^2$ and $R^3$, taken separately, are each independently $(C_1\text{-}C_4)$ alkyl; or taken together with the nitrogen to which they are attached form a pyrrolidine, piperidine or morpholine ring;

$R^4$ is hydrogen or methyl;

$R^5$ is $(C_1\text{-}C_6)$alkyl, $(C_1\text{-}C_6)$carboxyalkyl, carboxycyclohexyl or carboxyphenyl; and

$R^6$ is $(C_1\text{-}C_6)$ alkyl.

The expression "pharmaceutically acceptable acid addition salt" refers to addition salts with inorganic and organic acids such as HCl, $HNO_3$, $H_2SO_4$, $H_3PO_4$, $CH_3SO_3H$, $CH_3C_6H_4SO_3H$, $CH_3COOH$, fumaric acid, succinic acid and citric acid.

The expression "pharmaceutically acceptable cationic salt" refers to carboxylate salts where the cation is such as sodium, potassium, calcium, magnesium, ammonium or protonated benzathine (N,N′-di-benzylethylenediamine), choline, ethanolamine, diethanolamine, ethylenediamine, meglamine (N-methylglucamine), benethamine (N-benzylphenethylamine), piperazine or tromethamine (2-amino-2-hydroxymethyl-1,3-propanediol).

Because of their ease of preparation and valuable aldose reductase inhibitory activity, more preferred compounds of the formula (I) have $X^1$ as hydrogen or 5-chloro, $X^2$ as hydrogen, and $R^1$ as

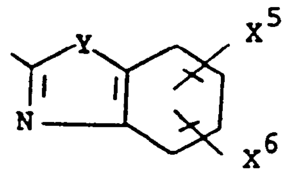

Most preferred compounds have Y as sulfur; and $X^1$ as 5-chloro, $X^5$ as hydrogen and $X^6$ as 5-fluoro; $X^1$, $X^5$ and $X^6$ each as hydrogen; $X^1$ as 5-chloro, $X^5$ as hydrogen and $X^6$ as 5-trifluoromethyl; $X^1$ as 5-chloro, $X^5$ as 5-fluoro and $X^6$ as 7-fluoro; or $X^1$ as hydrogen, $X^5$ as 5-fluoro and $X^6$ as 7-fluoro.

The present invention is also directed to pharmaceutical compositions for the control of chronic diabetic complications in mammals which comprise a compound of the formula (I) in a pharmaceutically acceptable carrier; and to their use in controlling chronic diabetic complications in a mammal suffering from chronic diabetes.

A further subject of the present invention is directed to the use of a compound of the formula II below for the manufacture of a medicament for controlling chronic diabetic complications in a mammal suffering from chronic diabetes:

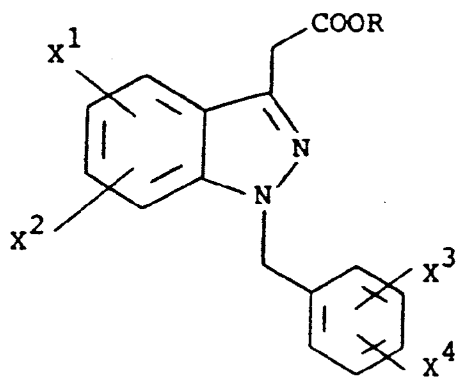

---(II)

wherein R, $X^1$, $X^2$, $X^3$ and $X^4$ are as defined above.

The aldose reductase inhibiting compounds (I) and (II) of the present invention are readily prepared according to the following scheme, in which R′ is R (as defined above), $(C_1-C_4)$alkyl, phenyl or benzyl; R″ is $R^1$ or $-C_6H_3X^3X^4$ (both as defined above); and X is a leaving group susceptible to nucleophilic displacement:

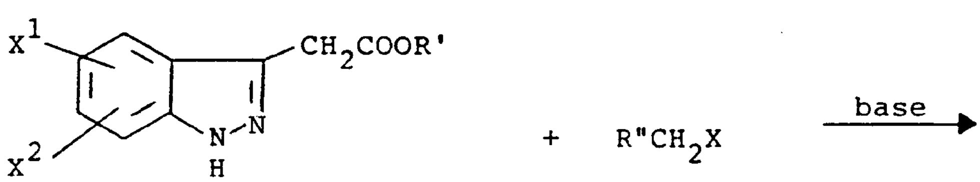

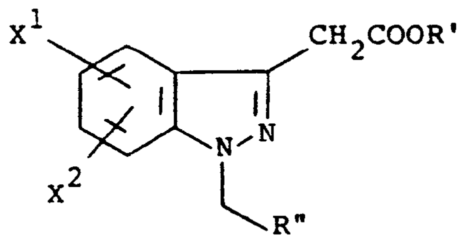

---(III)*

*(I) when R' is R and R" is $R^1$

(II) when R' is R and R" is $-C_6H_3X^3X^4$

when R' is H; conventional esterification

when R' is $(C_1-C_4)$-alkyl, phenyl or benzyl; conventional hydrolysis

(I) or (II) wherein R is a radical forming an ester hydrolyzable under physiological conditions

(I) or (II) wherein R is H

The displacement of X in the compound $R''CH_2X$ to form a compound of the formula (III) is a typical nucleophilic displacement reaction, generally carried out in a reaction inert solvent in the presence of a base. For example, the group X can be chloro, bromo, iodo, mesyl ($-SO_3CH_3$) or tosyl. For best results, the reaction is carried out on the anionic form of the 1H-indazole, readily obtained in situ by the action of a strong base on the free indazole. When R′ is H, hydrous conditions are satisfactory, such that even KOH or NaOH are useful for this purpose. In this case, at least 2 molar equivalents (and usually an excess, e.g., 3-molar equivalents) of base are used, in order to form the dianion and assure selective reaction at nitrogen. However, when R is a radical forming an ester, anhydrous and (except when the solvent is R′OH) aprotic conditions are much preferred, so as to avoid undesired hydrolysis and/or ester exchange. Thus, in the latter case, sodium hydride (or where practical R′ONa) is the preferred base for prior formation of the anion. In this case, only one molar equivalent of the base is required, with no more than modest excesses (e.g., 0.1 molar equivalent) generally employed. Solvent is not critical in this nucleophilic displacement, except to generally avoid protic solvents other than R′OH (e.g., aqueous solvents are fully satisfactory when R′ is H, methanolic solvents are satisfactory when R′ is $CH_3$, etc.). In any case, the solvent should be significantly less acidic than the indazole so as to maintain the latter in anionic form. Temperature is likewise not critical, e.g., temperature ranges of 0-100°C are generally satisfactory, temperatures at or near ambient being most convenient. For example, when X is bromo, the displacement will generally be complete within 15-30 minutes using a modest molar excess of the organic bromide to force the reaction to completion. Of course, when X is chloro, more time will be needed, while when X is iodo, less time will be needed. If desired, when X is other than iodo, the displacement reaction can be catalyzed by use of up to a mol or more of an iodide salt (e.g., NaI, KI).

As used here and elsewhere herein, the expression "reaction-inert solvent" refers to a solvent which does not interact with starting material, reagents, intermediates or desired product in a manner which adversely affects the yield of the desired product.

When R′ corresponds to R as defined above, the desired product (I) or (II) is of course directly formed in the displacement reaction. On the other hand, when R′ is $(C_1-C_4)$alkyl, phenyl or benzyl, the ester will then be conventionally hydrolyzed to form the compound of the formula (I) or (II) wherein R is hydrogen. Preferred are basic conditions, for example, using at least one molar equivalent (and usually an excess) of an aqueous alkali metal hydroxide, generally in the presence of a reaction-inert, water miscible organic solvent to aid in solubilizing the ester.

When in the product is (I) or (II) wherein R is hydrogen, and an ester hydrolyzable under physiological conditions is desired, such esters are also prepared according to usual methods. Thus when R is $-CH_2CH_2NR^2R^3$

the esters are readily prepared by reacting an activated form of the acid with a 2-(substituted-amino)ethanol of the formula

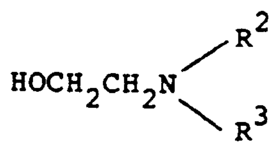 ---(IV)

Mixed anhydrides are well-suited as the activated form of the acid in such preparations. Generally, the acids are first converted in situ to a tertiary amine salt in the presence of a 1 to 1.1 molar excess of the amine. A variety of tertiary amines are suitable for this purpose. Exemplary are triethylamine, N-methylpiperidine, N-methylmorpholine, dimethylaniline or quinoline. Suitable inert solvents are methylene chloride, chloroform, dimethylformamide, and dimethylacetamide. It is preferrable that the acid be completely dissolved by the excess of tertiary amine, which may require a stirring period, together with gentle warming, if necessary. The solution of amine salt is then reacted with an equivalent of alkyl (e.g. ethyl), benzyl, or phenyl chloroformate, at a temperature in the range of -40° to 25°C., preferably in the range -10° to 10°C., to form a mixed anhydride in solution. Without isolation, the mixed anhydride is reacted directly with the appropriate alcohol of the formula (IV) to yield the desired ester. The reaction is usually initiated at a cool temperature (such as -40° to 15°C.), but allowed to warm to higher temperature (such as 15° to 40°C.) to complete the reaction. Alternatively, such esters are prepared by ester exchange, as specifically exemplified below. Thus, an intermediate ester of the formula (III) wherein R′ is ($C_1$-$C_4$)alkyl, phenyl or benzyl is reacted with an excess of the sodium salt of the amino alcohol (IV). The latter is generally formed in situ by reaction of the amino alcohol with NaH, in a reaction-inert solvent such as toluene, usually at a temperature in the range of 15-85°C.

The esters wherein R is a conventional radical forming an ester which is hydrolyzable under physiological conditions are more generally prepared by reaction of a salt of the acid (I or II, R=H; preferably the tetrabutylammonium salt) with an appropriate compound containing a displaceable halide (iodide, bromide or chloride; generally preferred, where available, in that order), or another group suitable for nucleophilic displacement. Exemplary are $CH_3OSO_2CH_3$, $C_2H_5Br$, $CH_3CH_2CH_2I$, $ICHR^4OCOR^5$, $ICHR^4OCOOR^6$,

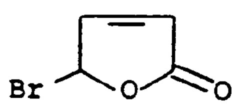 ,

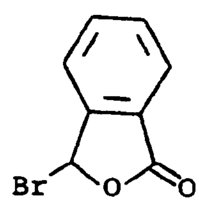 and

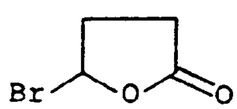 .

The required salt can be in isolated form, or more conveniently, formed in situ from the acid by use of at least one equivalent of a base. The reaction is carried out in a reaction-inert solvent, preferably one which is essentially anhydrous. A particularly convenient reaction system employs excess potassium carbonate as base in acetone as solvent. When the halide is chloro or bromo, up to three or more equivalents of anhydrous sodium iodide is added, if desired, to enhance the rate of reaction. An excess of the halide reagent is not critical to the reaction, but such an excess will generally be used in order to force the reaction to completion in a shorter period of time. The rate of reaction will also depend greatly on the halide (e.g., I > Br > Cl) and on the nature of the radical group R (e.g., more branched $ICHCH_3OCOCH_3$ will react more slowly than $ICH_2OCOCH_3$). The reaction temperature is not critical, a temperature in the range of 0-100°C. being generally satisfactory, but ambient or near ambient temperatures are preferred. Another preferred method converts the free acid form into the tetrabutylammonium salt, formed in water and extracted into an organic solvent such as chloroform, which is then reacted with the organic halide. A typical procedure employing the latter method is exemplified below.

By conventional modification of the isolation procedure, the compounds of the formula (I) and (II) are alternatively isolated in the form of a pharmaceutically-acceptable acid addition salt, as defined above. Such salts are also readily prepared from the isolated free base forms by standard methods. For example, a molar equiv-

alent of HCl, HBr, $HNO_3$ or succinic acid, or a half a molar equivalent of $H_2SO_4$ or succinic acid is combined with the free base in an organic or aqueous solvent to form the HCl, HBr, $HNO_3$, hemisuccinate, bisulfate or succinate salt, respectively. The salt is isolated by concentration and/or the addition of a non-solvent.

Similarly, by modification of the isolation procedure, the compounds of the formula (I) or (II) wherein R is H are alternatively isolated in the form of a pharmaceutically-acceptable cationic salt, as defined above. Such salts are also readily prepared from the isolated acid forms by standard methods. For example, an equivalent of the corresponding cationic hydroxide, carbonate or bicarbonate, or of an amine, is combined with the carboxylic acid in an organic or aqueous solvent. The salt is isolated by concentration and/or the addition of a non-solvent.

The 1H-indazole-3-acetic acids generally required as starting materials for synthesis of the compounds of the present invention are readily available according to literature methods, for example, by chain extension of an extensive variety of 1H-indazole-3-carboxylic acids described in above cited Corsi et al., using the method also there described:

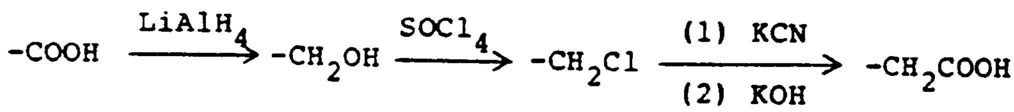


Corresponding ($C_1$-$C_4$)alkyl, benzyl or phenyl 1H-indazole-3-acetate esters are best prepared by conventional acid catalyzed esterification, as exemplified in the preparations below. The same can be applied to the preparation of 1H-indazole-3-acetate esters of aminoethanols of the formula (IV), which are alternatively prepared by the ester exchange method exemplified below. It will be evident to those skilled in the art that any such esters which are prepared via activated forms of the acid, or via nucleophilic displacement will generally require protection of the indazole nitrogen, e.g., by means of a benzyloxycarbonyl group removable by hydrogenolysis once the ester group is in place. As a practical matter, it is therefore usually preferred to introduce such ester groups after the benzyl or heteroarylmethyl group is already in place.

Variously substituted benzyl halides and heteroarylmethyl halides also required as starting materials are also readily available, in many cases commercially, but in any event by conventional methods, for example, from corresponding carboxylate esters, carboxylic acids, aldehydes, and carbinols, e.g.

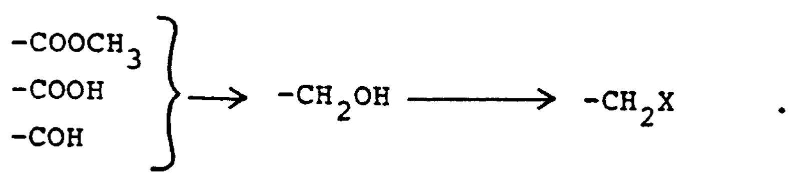

The present compounds of the formulas (I) and (II), particularly in their acid or salt form, are tested in vitro for their ability to reduce or inhibit aldose reductase enzyme activity, following the procedure described in U.S. Patent No. 3,821,383 and based on the procedure of Hayman et al., Journal of Biological Chemistry, 240, 877 (1965). The substrate employed is partially purified aldose reductase enzyme obtained from human placenta. The results obtained with each compound at a concentration of $10^{-5}$M or lower are expressed as percent inhibition of enzyme activity, or, when tested at several concentration levels, expressed as an $IC_{50}$, the inhibition concentration calculated to show 50% inhibition of enzyme activity.

The present compounds of the formulas (I) and (II) are tested in vivo for their ability to reduce or inhibit sorbitol accumulation in the sciatic nerve of streptozotocinized (i.e., diabetic) rats by a procedure essentially as described in U.S. Patent No. 3,821,383. In the present study, the amount of sorbitol accumulation in the sciatic nerves was measured 27 hours after induction of diabetes. The compounds are generally administered orally at doses ranging from 2.5 to 100 mg/kg at 4, 8 and 24 hours following the administration of streptozotocin. The results obtained in this manner are presented in terms of percent inhibition afforded by the test compound as compared to the case where no compound was administered (i.e., the untreated animal where sorbitol levels normally rise from approximately 50-100 mM/g tissue to as high as 400 mM/g tissue during the test period). In this test values below 20% are not always experimentally and statistically significant. Not all of the compounds of the present invention show in vivo activity by this oral test. Such compounds will find parenteral or topical use as described below.

The compounds of this invention are all readily adapted to therapeutic use as aldose reductase inhibitors for the control of chronic diabetic complications in mammals. They are administered either orally or parenterally, or topically as eye drops, in dosages ranging from about 0.1 to 10 mg/kg of body weight per day in single or divided doses. Of course, in particular situations, at the discretion of the attending physician, doses outside of this range will be used.

The compounds of this invention can be administered in a wide variety of different dosage forms, i.e., they may be combined with various pharmaceutically-acceptable inert carriers in the form of tablets, capsules, lozenges, troches, hard candies, powders, sprays, elixirs, syrups, injectable or eye drop solutions, and the like. Such carriers include solid diluents or fillers, sterile aqueous media and various non-toxic organic solvents.

For purposes of oral administration, tablets containing various excipients such as sodium citrate, calcium carbonate and calcium phosphate are employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the essential active ingredient therein can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble, alkali metal or alkaline-earth metal salts previously enumerated. Such aqueous solutions should be suitably buffered, if necessary, and the liquid diluent first rendered isotonic with sufficient saline or glucose. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of topical administration, dilute, sterile, aqueous solutions (usually in about 0.1% to 5% concentration), otherwise similar to the above parenteral solutions, are prepared in containers suitable for dropwise administration to the eye.

The present invention is illustrated by the following examples.

## EXAMPLE 1

### 1-[(benzothiazol-2-yl)methyl]-1H-indazole-3-acetic acid

To a vigorously stirring solution of 1H-indazole-3-acetic acid (0.88 g) in water (100 ml) containing sodium hydroxide (0.60 g) was added 2-(bromomethyl)benzothiazole (1.25 g) and the resulting mixture heated at 80°C for 2.5 hours. The reaction solution was cooled to room temperature and extracted with ether (2 x 25 ml). The aqueous layer was collected and acidified to a pH of about 4.0 with concentrated HCl, and then extracted with ethyl acetate (2 x 20 ml). The organic layers were combined, dried and evaporated to a yellow solid (yield: 0.67 g), which was crystallized from benzene (m.p. 164-165°C).

Substituting a molar equivalent of 5-chloro-1H-indazole-3-acetic acid for 1H-indazole-3-acetic acid, the same method was employed to prepare:

1-(benzothiazol-2-yl)methyl-5-chloro-1H-indazole-3-acetic acid (m.p. 213°C).

Further substituting a molar equivalent of 3-(bromomethyl)benzo[d]isothiazole, 2-(bromomethyl)benzoxazole or 2-bromomethylquinoline for the 2-(bromomethyl)benzothiazole, the same method was used to prepare:

1-[(benzo[d]isothiazol-3-yl)methyl]-5-chloro-1H-indazole-3-acetic acid (m.p. 204-205°C);
1-[(benzoxazol-2-yl)methyl]-5-chloro-1H-indazole-3-acetic acid (m.p. 194-195°C); and
1-[(2-quinolyl)methyl]-5-chloro-1H-indazole-3-acetic acid (m.p. 201-202°C).

## EXAMPLE 2

### Methyl 1-(4-bromo-2-fluorobenzyl)-1H-indazole-3-acetate

To a solution obtained by adding sodium hydride (0.14 g; 50% w/w dispersion in mineral oil) to dimethylformamide (3 ml) containing methyl 1H-indazole-3-acetate (0.45 g) was added 4-bromo-2-fluorobenzyl bromide

(0.70 g). After 15 minutes, the reaction solution was poured onto ice water (20 ml). Sufficient 10% HCl was added to adjust the pH to about 4.0 and the solution then extracted with ethyl acetate (2 x 20 ml). The combined organic extract was washed with water (2 x 20 ml), dried and evaporated. The residue was purified by chromatography on silica gel [yield: 0.31 g; $^1H$ NMR ($CDCl_3$) 3.6 (s, 3H), 4.0 (s, 2H), 5.4 (s, 2H), 6.8-7.2 (m, 6H), 7.6 (m, 1H)].

Substituting a molar equivalent of 2-(bromomethyl)-5-(trifluoromethyl)benzothiazole, 2-(bromomethyl)-5-fluorobenzothiazole, 2-(bromomethyl)benzothiophene, 2-(bromomethyl)-5-(trifluoromethyl)benzoxazole or 2-(bromomethyl)-5,7-difluorobenzthiazole, respectively for 4-bromo-2-fluorobenzyl bromide, the same method was used to prepare:

methyl 1-[(5-(trifluoromethyl)benzothiazol-2-yl)methyl]-1H-indazole-3-acetate [$^1H$ NMR ($CDCl_3$, 60MHz): 3.65 (s, 3H), 4.0 (s, 2H), 5.9 (s, 2H), 7.0-8.2 (m, 7H)];

methyl 1-[(5-fluorobenzothiazol-2-yl)methyl]-1H-indazole-3-acetate [$^1H$ NMR ($CDCl_3$, 60 MHz): 3.6 (s, 3H), 4.05 (s, 2H), 5.9 (s, 2H), 6.9-7.9 (m, 7H)];

methyl 1-[(benzothien-2-yl)methyl]-1H-indazole-3-acetate [$^1H$ NMR ($CDCl_3$, 60MHz): 3.65 (s, 3H), 4.05 (s, 2H), 4.65 (s, 2H), 7.0-7.8 (m, 9H)];

methyl 1-[(5-(trifluoromethyl)benzoxazol-2-yl)methyl]-1H-indazole-3-acetate [$^1H$NMR ($CDCl_3$, 60MHz); 3.50 (s, 3H), 4.05 (s, 2H), 4.8 (s, 2H), 7.0-7.9 (m, 7H)]; and

methyl 1-[(5,7-difluorobenzothiazol-2-yl)methyl]-1H-indazole-3-acetate [$^1H$NMR ($CDCl_3$, 60MHz); 1.3 (t, J=8Hz, 3H), 4.0 (s, 2H), 4.15 (q, J=8Hz, 2H), 5.9 (s, 2H), 6.9 (m, 1H), 7.2-7.7 (m, 5H).

Further substituting a molar equivalent of the corresponding ethyl ester for methyl 1H-indazole-3-acetate, and a molar equivalent of 2-(bromomethyl)-6-bromobenzothiazole, 2-(bromomethyl-5-chlorobenzoxazole or 5-(bromomethyl)-3-(2-bromophenyl)-1,2,4-oxadiazole, respectively, for 4-bromo-2-fluorobenzyl bromide, the same method was used to prepare:

ethyl 1-[(6-bromobenzothiazol-2-yl)methyl]-1H-indazole-3-acetate [$^1H$NMR $CDCl_3$, 60MHz): 1.25 (t, J=8Hz, 3H), 4.05 (s, 2H), 4.2 (q, J=8Hz, 2H), 5.9 (s, 2H), 7.1-7.9 (m, 7H)];

ethyl 1-[(5-chlorobenzoxazol-2-yl)methyl]-1H-indazole-3-acetate [$^1H$NMR ($CDCl_3$, 60MHz): 1.2 (t, J=8Hz, 3H), 4.0 (s, 2H), 4.1 (q, J=8Hz, 2H), 5.8 (s, 2H), 7.0-7.8 (m, 7H)]; and

ethyl 1-[(3-(2-bromophenyl)-1,2,4-oxadiazol-5-yl)methyl]-1H-indazole-3-acetate [$^1H$NMR ($CDCl_3$, 60MHz): 1.2 (t, J=8Hz, 3H), 4.0 (s, 2H), 4.2 (q, J=8Hz, 2H), 5.8 (s, 2H), 7.1-7.7 (m, 8H)].

Further substituting a molar equivalent of ethyl 5-chloro-1H-indazole-3-acetate for the methyl 1H-indazole-3-acetate, and 2-(bromomethyl)-5-bromobenzothiazole, 2-(bromomethyl)-5-chlorobenzoxazole, 2-(bromomethyl)-5-fluorobenzothiazole and 2-(bromomethyl)-5-(trifluoromethyl)benzothiazole, respectively, for the 4-bromo-2-fluorobenzyl bromide, the same method was used to prepare:

ethyl 1-[(5-bromobenzothiazol-2-yl)methyl]-5-chloro-1H-indazole-3-acetate. $^1H$NMR ($CDCl_3$, 60MHz): 1.30 (t, J=8Hz, 3H), 4.0 (s, 2H), 4.2 (q, J=8Hz, 2H), 5.85 (s, 2H), 7.1 (m, 4H), 7.6 (dd, J=2,7Hz, 1H), 8.05 (d, J=2Hz, 1H)];

ethyl 1-[(5-chlorobenthiazol-2-yl)methyl-5-chloro-1H-indazole-3-acetate [$^1H$NMR ($CDCl_3$, 60MHz): 1.25 (t, J=8Hz, 3H), 3.95 (s, 2H), 4.1 (q, J=8Hz, 2H), 5.65 (s, 2H), 7.1 (m, 5H), 7.5 (m, 2H)];

ethyl 1-[(5-fluorobenthiazol-2-yl)methyl-5-chloro-1H-indazole-3-acetate [$^1H$NMR ($CDCl_3$, 60MHz): 1.3 (t, J=8Hz, 3H), 4.1 (s, 2H), 4.2 (q, J=8Hz, 2H), 5.95 (s, 2H), 7.1-7.9 (m, 6H)]; and

ethyl 1-[(5-trifluoromethyl)benzothiazol-2-yl)methyl]-5-chloro-1H-indazole-3-acetate [$^1H$NMR ($CDCl_3$, 60MHz): 1.25 (t, J=8Hz, 3H), 4.05 (s, 2H), 4.2 (q, J=8Hz, 2H), 5.9 (s, 2H), 7.1-8.2 (m, 6H)].

Further substituting a molar equivalent of methyl 5-chloro-1H-indazole-3-acetate for the methyl 1H-indazole-3-acetate, and 2-(bromomethyl)-5,7-difluorobenzothiazole for the 4-bromo-2-fluorobenzyl bromide, the same method was used to prepare:

methyl 5-chloro-1-[(5,7-difluorobenzothiazol-2-yl)methyl]-1H-indazole-3-acetate [m.p. 109-112°C].

## EXAMPLE 3

### 1-(4-bromo-2-fluorobenzyl)-1H-indazole-3-acetic acid

A solution of methyl 1-(4-bromo-2-fluorobenzyl)-1H-indazole-3-acetate (0.30 g) in methanol (5 ml) containing 10% aqueous KOH (1 ml) was stirred at room temperature for 16 hours. It was then concentrated to a low volume, and then diluted with ethyl acetate (10 ml) and sufficient 10% HCl added to adjust the pH to about 4.0. The ethyl acetate layer was washed with water (5 ml), dried and then evaporated to a white solid (yield: 0.21 g; m.p. 167-168°C).

By the same method, the other methyl and ethyl esters of the proceding Example were converted to:

1-[(5-(trifluoromethyl)benzothiazol-2-yl)methyl]-1H-indazole-3-acetic acid (m.p. 168-169°C);

1-[(5-fluorobenzothiazol-2-yl)methyl]1H-indazole-3-acetic acid (m.p. 173-174°C);
1-[(benzothien-2-yl)methyl]-1H-indazole-3-acetic acid (m.p. 164-165°C);
1-[(5-(trifluoromethyl)benzoxazol-2-yl)methyl]-1H-indazole-3-acetic acid (m.p. 204-205°C);
1-[(5,7-difluorobenzothiazol-2-yl)methyl]-1H-indazole-3-acetic acid (m.p. 168-169°C);
1-[(6-bromobenzothiazol-2-yl)methyl]-1H-indazole-3-acetic acid (m.p. 186-189°C);
1-[(5-chlorobenzoxazol-2-yl)methyl]-1H-indazole-3-acetic acid (m.p. 197-198°C);
1-[(3-(2-bromophenyl)-1,2,4-oxadiazol-5-yl)methyl]-1H-indazole-3-acetic acid (m.p. 208-209°C);
1-[(5-bromobenzothiazol-2-yl)methyl]-5-chloro-1H-indazole-3-acetic acid (m.p. 210-211°C);
1-[(5-chlorobenzoxazol-2-yl)methyl-5-chloro-1H-indazole-3-acetic acid (m.p. 227-228°C);
1-[(5-fluorobenzothiazol-2-yl)methyl-5-chloro-1H-indazole-3-acetic acid (m.p. 186-188°C); and
1-[(5-(trifluoromethyl)benzothiazol-2-yl)methyl]-5-chloro-1H-indazole-3-acetic acid (m.p. 189-190°C);
1-[(5,7-difluorobenzothiazol-2-yl)methyl]-5-chloro-1H-indazole-3-acetic acid (m.p. 196°C).

PREPARATION 1

Methyl 1H-indazole-3-acetate

A solution of 1H-indazole-3-acetic acid (1.0 g) in methanol (30 ml) containing five drops of concentrated sulfuric acid was refluxed for 8 hours. It was then concentrated to a low volume, diluted with ethyl acetate (20 ml). The organic layer washed with water (2 x 10 ml) and then with sodium bicarbonate solution (10 ml, 10%). The ethyl acetate layer was collected and then dried to obtain the title compound (yield: 0.8 g; m.p. 146°C).

The corresponding ethyl ester was prepared by substituting anhydrous ethanol for methanol.

## Claims

**Claims for the following Contracting States : AT, BE, CH, DE, FR, GB, IT, LI, LU, NL, SE**

1. A compound of the formula

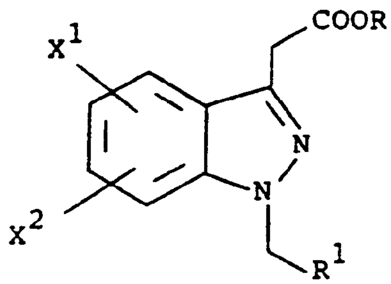

---(I)

wherein

$X^1$ and $X^2$ are each independently hydrogen, fluoro, chloro, bromo, trifluoromethyl, $(C_1\text{-}C_3)$alkyl or $(C_1\text{-}C_3)$alkoxy;

R is hydrogen or a radical group forming a conventional ester which is hydrolyzable under physiological conditions;

$R^1$ is

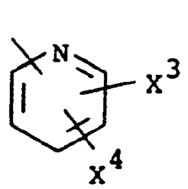 , 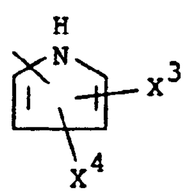 , 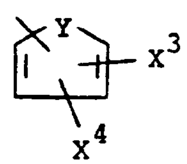 ,

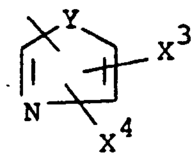 , 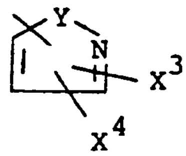 , 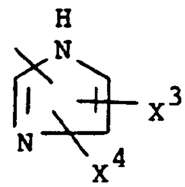 ,

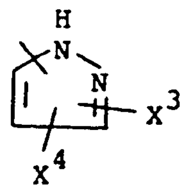 or 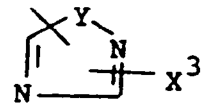 ;

Y is sulfur or oxygen;

$X^3$, when taken separately, is hydrogen, fluoro, chloro, bromo, trifluoromethyl, ($C_1$-$C_3$)alkyl, ($C_1$-$C_3$)alkoxy or

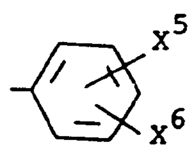 ;

$X^4$, when taken separately, $X^5$ and $X^6$ are each independently hydrogen, fluoro, chloro, bromo, trifluoromethyl, ($C_1$-$C_3$)alkyl or ($C_1$-$C_3$)alkoxy; and

$X^3$ and $X^4$, when taken together, are combined with the adjacent carbons to which they are attached to form a benzene ring substituted by $X^5$ and $X^6$;

a pharmaceutically acceptable cationic salt thereof when R is hydrogen; or

a pharmaceutically acceptable acid addition salt thereof.

2. A compound of claim 1 wherein R is:

1H-furan-5-on-1-yl;

1H-isobenzofuran-3-on-1-yl;

gamma-butyrolacton-4-yl;

$-CH_2CH_2NR^2R^3$;

$-CHR^4OCOR^5$; or

$-CHR^4OCOOR^6$;

wherein

$R^2$ and $R^3$, taken separately, are each independently ($C_1$-$C_4$)alkyl; or taken together with the nitrogen to which they are attached form a pyrrolidine, piperidine or morpholine ring;

$R^4$ is hydrogen or methyl;

$R^5$ is ($C_1$-$C_6$)alkyl, ($C_1$-$C_6$)carboxyalkyl, carboxycyclohexyl or carboxyphenyl; and

$R^6$ is ($C_1$-$C_6$)alkyl.

3. A compound of claim 1 wherein R is hydrogen.

4. A compound of claim 3 wherein $X^1$ is hydrogen or 5-chloro, and $X^2$ is hydrogen.

5. A compound of claim 4 wherein $R^1$ is

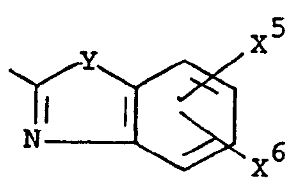 .

6. A compound of claim 5 wherein Y is sulfur.

7. A compound of claim 6 wherein $X^1$ is 5-chloro, $X^5$ is hydrogen and $X^6$ is 5-fluoro or 5-trifluoromethyl.

8. The compound of claim 6 wherein $X^1$, $X^5$ and $X^6$ are each hydrogen.

9. A compound of claim 6 wherein $X^5$ is 5-fluoro and $X^6$ is 7-fluoro.

10. A pharmaceutical composition comprising a compound of the formula (I) as claimed in any one of claims 1 to 9 in combination with a pharmaceutically acceptable diluent or carrier.

11. The use of a compound of the formula (I) as claimed in any one of claims 1 to 9, or a compound of the formula:

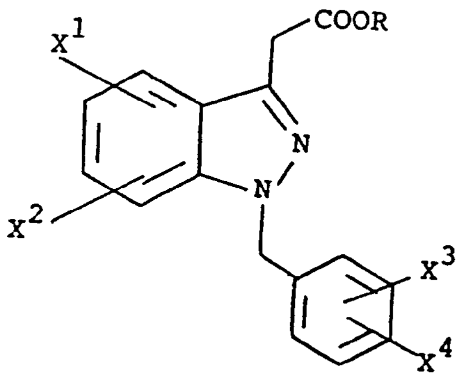

---(II)

wherein R, $X^1$, $X^2$, $R^3$ and $X^4$ are as defined in claim 1, for the manufacture of a medicament for controlling diabetic complications in a mammal suffering from chronic diabetes.

**Claims for the following Contracting States : ES, GR**

1. A process for a compound of the formula

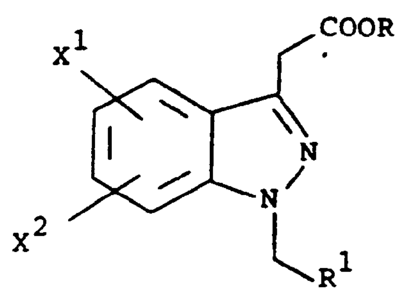

---(I)

wherein

$X^1$ and $X^2$ are each independently hydrogen, fluoro, chloro, bromo, trifluoromethyl, $(C_1\text{-}C_3)$alkyl or $(C_1\text{-}C_3)$alkoxy;

R is hydrogen or a radical group forming a conventional ester which is hydrolyzable under physiological conditions;

$R^1$ is

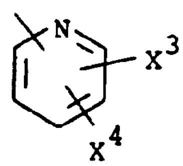 , 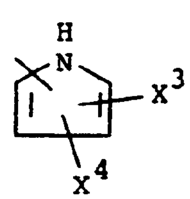 , 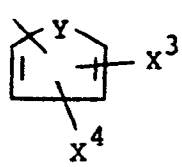 ,

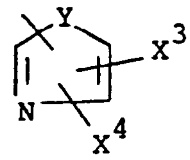 , 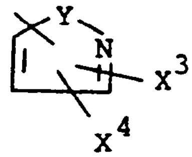 , 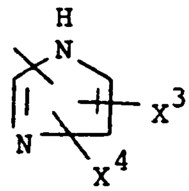 ,

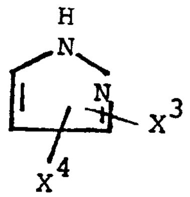 or 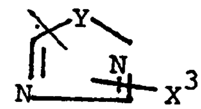 ;

Y is sulfur or oxygen;

$X^3$, when taken separately, is hydrogen, fluoro, chloro, bromo, trifluoromethyl, $(C_1\text{-}C_3)$alkyl, $(C_1\text{-}C_3)$alkoxy or

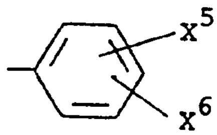 ;

$X^4$, when taken separately, $X^5$ and $X^6$ are each independently hydrogen, fluoro, chloro, bromo, trifluoromethyl, $(C_1\text{-}C_3)$alkyl or $(C_1\text{-}C_3)$alkoxy; and

$X^3$ and $X^4$, when taken together, are combined with the adjacent carbons to which they are attached to form a benzene ring substituted by $X^5$ and $X^6$;

a pharmaceutically acceptable cationic salt thereof when R is hydrogen; or

a pharmaceutically acceptable acid addition salt thereof, which comprises

(a) reaction of a compound of the formula

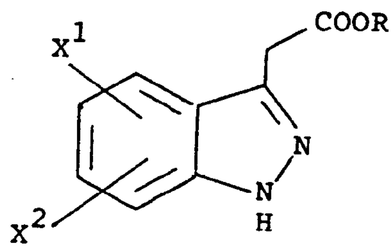

---(IV)

with a compound of the formula

$R^1\text{-}CH_2\text{-}X$,

wherein R, $R^1$, $X^1$ and $X^2$ are as defined above and X is a leaving group susceptible to nucleophilic displacement, in a reaction inert solvent in the presence of a base; and if desired,

(b) when R is hydrogen, conventional solvolysis of a preformed ester compound of the formula (I) wherein R is other than hydrogen;

(c) when R is other than hydrogen, conventional esterification of a preformed acid compound of the formula (I) wherein R is hydrogen;

(d) when R is hydrogen, conventional conversion to a pharmaceutically acceptable cationic salt; or

(e) conventional conversion to an acid addition salt.

2. A process of claim 1 wherein R is:

1H-furan-5-on-1-yl;

1H-isobenzofuran-3-on-1-yl;

gamma-butyrolacton-4-yl;

$-CH_2CH_2NR^2R^3$;
$-CHR^4OCOR^5$; or
$-CHR^4OCOOR^6$;

wherein

$R^2$ and $R^3$, taken separately, are each independently ($C_1$-$C_4$)alkyl; or taken together with the nitrogen to which they are attached form a pyrrolidine, piperidine or morpholine ring;

$R^4$ is hydrogen or methyl;

$R^5$ is ($C_1$-$C_6$)alkyl, ($C_1$-$C_6$)carboxyalkyl, carboxycyclohexyl or carboxyphenyl; and

$R^6$ is ($C_1$-$C_6$)alkyl.

3. A process of claim 1 wherein R is hydrogen.

4. A process of claim 3 wherein $X^1$ is hydrogen or 5-chloro, and $X^2$ is hydrogen.

5. A process of claim 4 wherein $R^1$ is

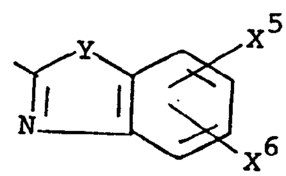

.

6. A process of claim 5 wherein Y is sulfur.

7. A process of claim 6 wherein $X^1$ is 5-chloro, $X^5$ is hydrogen and $X^6$ is 5-fluoro or 5-trifluoromethyl.

8. A process of claim 6 wherein $X^1$, $X^5$ and $X^6$ are each hydrogen.

9. A process of claim 6 wherein $X^5$ is 5-fluoro and $X^6$ is 7-fluoro.

10. A process for the preparation of a pharmaceutical composition for the control of chronic diabetic complications in mammals which comprises combining a compound of claim 1 or a compound of the formula

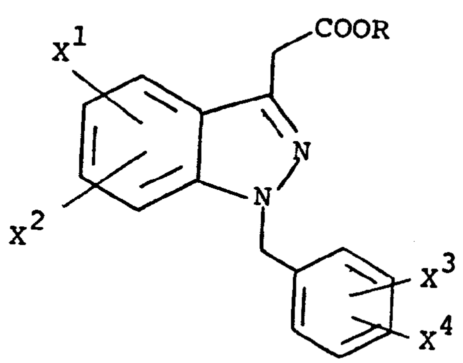

---(II)

,

wherein R, $X^1$, $X^2$, $X^3$ and $X^4$ are as defined in claim 1,

a pharmaceutically acceptable cationic salt thereof when R is hydrogen; or

a pharmaceutically acceptable acid addition salt thereof;

with a pharmaceutically acceptable carrier.

**Patentansprüche**

**Patentansprüche für folgende Vertragsstaaten : AT, BE, CH, DE, FR, GB, IT, LI, LU, NL, SE**

1. Verbindung der Formel

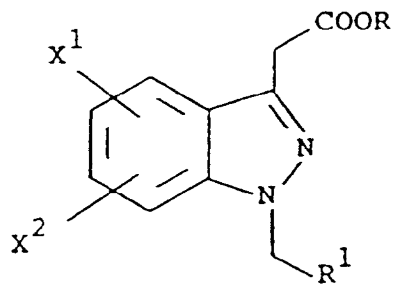

(I),

worin

$X^1$ und $X^2$ jeweils unabhängig Wasserstoff, Fluor, Chlor, Brom, Trifluormethyl, $(C_1$-$C_3)$-Alkyl oder $(C_1$-$C_3)$-Alkoxy bedeuten;

R Wasserstoff oder einen Rest darstellt, der einen herkömmlichen Ester bildet, welcher unter physiologischen Bedingungen hydrolysierbar ist;

$R^1$

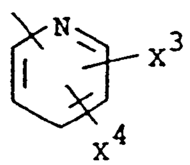

,

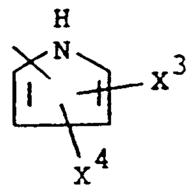

,

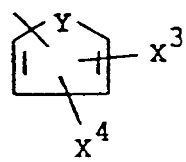

,

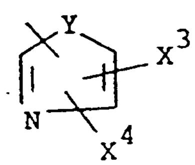

,

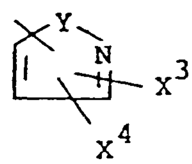

,

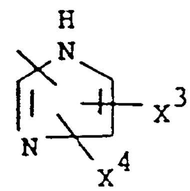

,

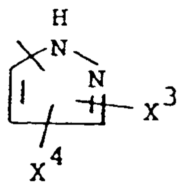

oder

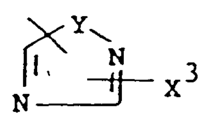

bedeutet;

Y Schwefel oder Sauerstoff ist;

$X^3$, wenn getrennt genommen, Wasserstoff, Fluor, Chlor, Brom, Trifluormethyl, $(C_1$-$C_3)$-Alkyl, $(C_1$-$C_3)$-Alkoxy oder

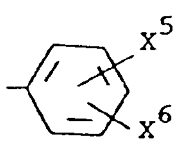

darstellt;

$X^4$, wenn getrennt genommen, $X^5$ und $X^6$ jeweils unabhängig Wasserstoff, Fluor, Chlor, Brom, Trifluormethyl, $(C_1$-$C_3)$-Alkyl oder $(C_1$-$C_3)$-Alkoxy sind, und

$X^3$ und $X^4$, wenn zusammen genommen, mit den benachbarten Kohlenstoffatomen, an die sie gebunden sind, unter Bildung eines durch $X^5$ und $X^6$ substituierten Benzolringes kombiniert sind,

ein pharmazeutisch annehmbares kationisches Salz hievon, wenn R Wasserstoff ist, und

ein pharmazeutisch annehmbares Säureadditionssalz hievon.

2. Verbindung nach Anspruch 1, in der R
1H-Furan-5-on-1-yl,
1H-isobenzofuran-3-on-1-yl,
y-Butyrolacton-4-yl,
$-CH_2CH_2NR^2R^3$,
$-CHR^4OCOR^5$ oder
$-CHR^4OCOOR^6$ bedeutet,

wobei

$R^2$ und $R^3$, getrennt genommen, unabhängig jeweils ($C_1$-$C_4$)-Alkyl bedeuten, oder zusammen genommen mit dem Stickstoff, an den sie gebunden sind, einen Pyrrolidin-, Piperidin- oder Morpholinring bilden,
$R^4$ Wasserstoff oder Methyl bedeutet,
$R^5$ ($C_1$-$C_6$)-Alkyl, ($C_1$-$C_6$)-Carboxyalkyl, Carboxycyclohexyl oder Carboxyphenyl darstellt und
$R^6$ ($C_1$-$C_6$)-Alkyl ist.

3. Verbindung nach Anspruch 1, in der R Wasserstoff ist.

4. Verbindung nach Anspruch 3, in der $X^1$ Wasserstoff oder 5-Chlor bedeutet und $X^2$ Wasserstoff ist.

5. Verbindung nach Anspruch 4, in der $R^1$

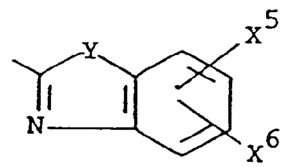

ist.

6. Verbindung nach Anspruch 5, in der Y Schwefel ist.

7. Verbindung nach Anspruch 6, in der $X^1$ 5-Chlor, $X^5$ Wasserstoff und $X^6$ 5-Fluor oder 5-Trifluormethyl bedeuten.

8. Verbindung nach Anspruch 6, in der $X^1$, $X^5$ und $X^6$ jeweils Wasserstoff bedeuten.

9. Verbindung nach Anspruch 6, in der $X^5$ 5-Fluor und $X^6$ 7-Fluor bedeuten.

10. Pharmazeutische Zusammensetzung, die eine Verbindung der Formel (I) nach einem der Ansprüche 1 bis 9 in Kombination mit einem pharmazeutisch annehmbaren Verdünnungsmittel oder Träger umfaßt.

11. Verwendung einer Verbindung der Formel (I) nach einem der Ansprüche 1 bis 9 oder einer Verbindung der Formel

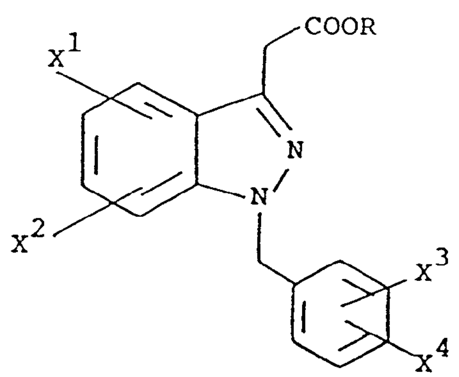

(II),

worin R, $X^1$, $X^2$, $X^3$ und $X^4$ wie in Anspruch 1 definiert sind, zur Herstellung eines Medikaments zum Beschränken diabetischer Komplikationen in einem Säuger, der an chronischem Diabetes leidet.

**Patentansprüche für folgende Vertragsstaaten : ES, GR**

1. Verfahren zum Herstellen einer Verbindung der Formel

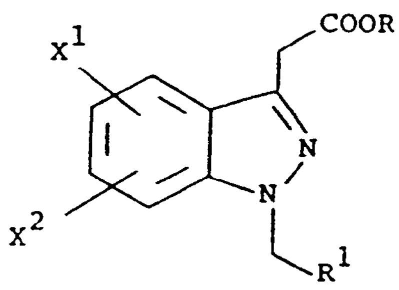

(I),

worin

$X^1$ und $X^2$ jeweils unabhängig Wasserstoff, Fluor, Chlor, Brom, Trifluormethyl, ($C_1$-$C_3$)-Alkyl oder ($C_1$-$C_3$)-Alkoxy bedeuten;

R Wasserstoff oder einen Rest darstellt, der einen herkömmlichen Ester bildet, welcher unter physiologischen Bedingungen hydrolysierbar ist;

$R^1$

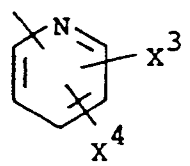 , 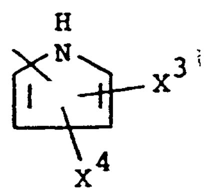 , 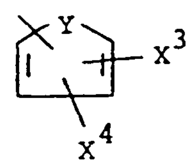 ,

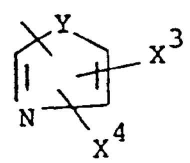 , 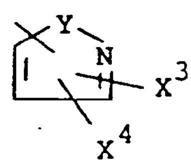 , 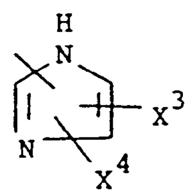 ,

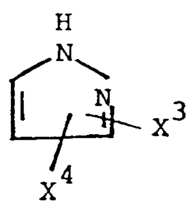 oder 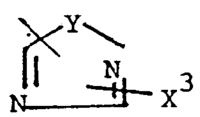

bedeutet;

Y Schwefel oder Sauerstoff ist;

$X^3$, wenn getrennt genommen, Wasserstoff, Fluor, Chlor, Brom, Trifluormethyl, ($C_1$-$C_3$)-Alkyl, ($C_1$-$C_3$)-Alkoxy oder

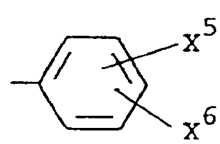

darstellt;

$X^4$, wenn getrennt genommen, $X^5$ und $X^6$ jeweils unabhängig Wasserstoff, Fluor, Chlor, Brom, Trifluormethyl, ($C_1$-$C_3$)-Alkyl oder ($C_1$-$C_3$)-Alkoxy sind, und

$X^3$ und $X^4$, wenn zusammen genommen, mit den benachbarten Kohlenstoffatomen, an die sie gebunden sind, unter Bildung eines durch $X^5$ und $X^6$ substituierten Benzolringes kombiniert sind,

eines pharmazeutisch annehmbaren kationisches Salzes hievon, wenn R Wasserstoff ist, oder

eines pharmazeutisch annehmbaren Säureadditionssalzes hievon, das umfaßt:

(a) die Reaktion einer Verbindung der Formel

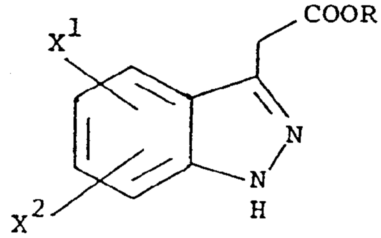

(IV)

mit einer Verbindung der Formel

$R^1$-$CH_2$-X ,

worin R, $R^1$, $X^1$ und $X^2$ wie oben definiert sind und X eine Abgangsgruppe darstellt, die für nukleophile Verdrängung geeignet ist, in einem reaktionsinerten Lösungsmittel in Anwesenheit einer Base und, wenn gewünscht,

(b) wenn R Wasserstoff ist, die herkömmliche Solvolyse einer vorgeformten Esterverbindung der Formel (I), worin R eine andere Bedeutung als Wasserstoff hat,

(c) wenn R eine andere Bedeutung als Wasserstoff hat, die herkömmliche Veresterung einer vorgeformten Säureverbindung der Formel (I), worin R Wasserstoff ist,

(d) wenn R Wasserstoff ist, die herkömmliche Überführung in ein pharmazeutisch annehmbares kationisches Salz oder

(e) die herkömmliche Überführung in ein Säureadditionssalz.

2. Verfahren nach Anspruch 1, in dem R

1H-Furan-5-on-1-yl,
1H-isobenzofuran-3-on-1-yl,
y-Butyrolacton-4-yl,
-$CH_2CH_2NR^2R^3$,
-$CHR^4OCOR^5$ oder
-$CHR^4OCOOR^6$

bedeutet, wobei

$R^2$ und $R^3$, getrenntgenommen, unabhängig jeweils ($C_1$-$C_4$)-Alkyl bedeuten, oder zusammen genommen mit dem Stickstoff, an den sie gebunden sind, einen Pyrrolidin-, Piperidin- oder Morpholinring bilden, $R^4$ Wasserstoff oder Methyl bedeutet, $R^5$ ($C_1$-$C_4$)-Alkyl, ($C_1$-$C_6$)-Carboxyalkyl, Carboxycyclohexyl oder Carboxyphenyl darstellt und $R^6$ ($C_1$-$C_6$)-Alkyl ist.

3. Verfahren nach Anspruch 1, in dem R Wasserstoff ist.

4. Verfahren nach Anspruch 3, in dem $X^1$ Wasserstoff oder 5-Chlor bedeutet und $X^2$ Wasserstoff ist.

5. Verfahren nach Anspruch 4, in dem $R^1$

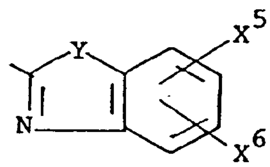

ist.

6. Verfahren nach Anspruch 5, in dem Y Schwefel ist.

7. Verfahren nach Anspruch 6, in dem $X^1$ 5-Chlor, $X^5$ Wasserstoff und $X^6$ 5-Fluor oder 5-Trifluormethyl bedeuten.

8. Verfahren nach Anspruch 6, in dem $X^1$, $X^5$ und $X^6$ jeweils Wasserstoff bedeuten.

9. Verfahren nach Anspruch 6, in dem $X^5$ 5-Fluor und $X^6$ 7-Fluor bedeuten.

10. Verfahren zur Herstellung einer pharmazeutischen Zusammensetzung für die Beschränkung chronischer diabetischer Komplikationen in Säugern, das das Vereinigen einer Verbindung nach Anspruch 1 oder einer Verbindung der Formel

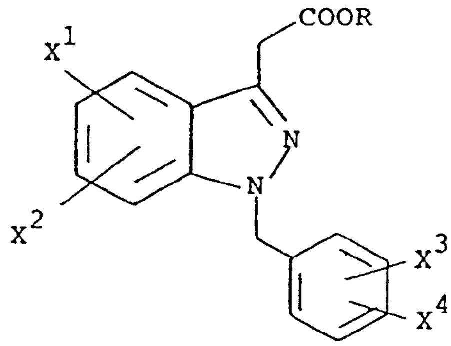

(II),

worin R, $X^1$, $X^2$, $X^3$ und $X^4$ wie in Anspruch 1 definiert sind, eines pharmazeutisch annehmbaren kationischen Salzes hievon, wenn R Wasserstoff ist, oder eines pharmazeutisch annehmbaren Säureadditionssalzes hievon mit einem pharmazeutisch annehmbaren Träger umfaßt.

## Revendications

### Revendications pour les Etats contractants suivants : AT, BE, CH, DE, FR, GB, IT, LI, LU, NL, SE

1. Composé de formule

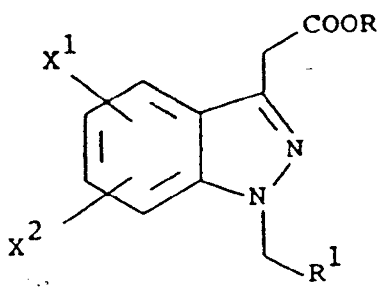

---(I)

dans laquelle

$X^1$ et $X^2$ représentent chacun, indépendamment, l'hydrogène, un radical fluoro, chloro, bromo, trifluorométhyle, alkyle en $C_1$ à $C_3$ ou alkoxy en $C_1$ à $C_3$ ;

R est l'hydrogène ou un radical formant un ester classique qui est hydrolysable dans des conditions physiologiques ;

$R^1$ représente

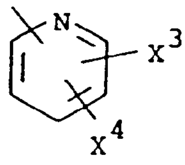 , 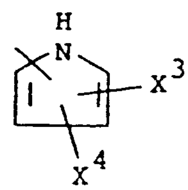 , 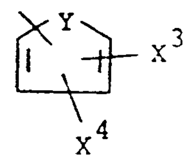 ,

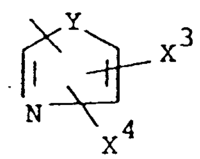 , 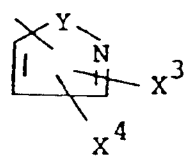 , 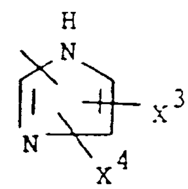 ,

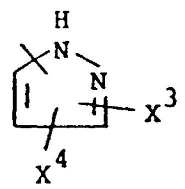 ou 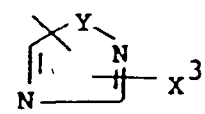 ;

Y représente le soufre ou l'oxygène ;

$X^3$, pris séparément, est l'hydrogène, un radical fluoro, chloro, bromo, trifluorométhyle, alkyle en $C_1$ à $C_3$, alkoxy en $C_1$ à $C_3$ ou

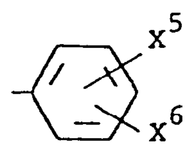 ;

$X^4$, pris séparément, $X^5$ et $X^6$ représentent chacun indépendamment l'hydrogène, un radical fluoro, chloro, bromo, trifluorométhyle, alkyle en $C_1$ à $C_3$ ou alkoxy en $C_1$ à $C_3$ ; et

$X^3$ et $X^4$, pris ensemble, conjointement avec les atomes adjacents de carbone auxquels ils sont attachés, forment un noyau benzénique portant les substituants $X^5$ et $X^6$ ;

un sel cationique pharmaceutiquement acceptable de ce composé lorsque R est l'hydrogène ; ou

un sel d'addition d'acide pharmaceutiquement acceptable de ce composé.

2. Composé suivant la revendication 1, dans lequel R représente un groupe :

1H-furanne-5-one-1-yle ;

1H-isobenzofuranne-3-one-1-yle ;

gamma-butyrolactone-4-yle ;

$-CH_2CH_2NR^2R^3$ ;

$-CHR^4OCOR^5$ ; ou

$-CHR^4OCOOR^6$ ;

où

$R^2$ et $R^3$, pris séparément, représentent chacun indépendamment un radical alkyle en $C_1$ à $C_4$ ; ou forment conjointement avec l'atome d'azote auquel ils sont attachés un noyau de pyrrolidine, pipéridine ou morpholine ;

$R^4$ est l'hydrogène ou un groupe méthyle ;

$R^5$ est un groupe alkyle en $C_1$ à $C_6$, carboxy(alkyle en $C_1$ à $C_6$), carboxycyclohexyle ou carboxyphényle ; et

$R^6$ est un groupe alkyle en $C_1$ à $C_6$.

3. Composé suivant la revendication 1, dans lequel R est l'hydrogène.

4. Composé suivant la revendication 3, dans lequel $X^1$ est l'hydrogène ou un radical 5-chloro et $X^2$ est l'hydrogène.

5. Composé suivant la revendication 4, dans lequel $R^1$ représente

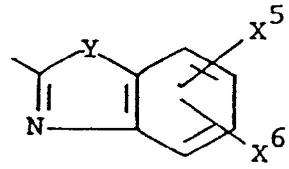

.

6. Composé suivant la revendication 5, dans lequel Y est le soufre.

7. Composé suivant la revendication 6, dans lequel $X^1$ est le radical 5-chloro, $X^5$ est l'hydrogène et $X^6$ est le radical 5-fluoro ou 5-trifluorométhyle.

8. Composé suivant la revendication 6, dans lequel $X^1$, $X^5$ et $X^6$ sont tous de l'hydrogène.

9. Composé suivant la revendication 6, dans lequel $X^5$ est le radical 5-fluoro et $X^6$ est le radical 7-fluoro.

10. Composition pharmaceutique comprenant un composé de formule (I) suivant l'une quelconque des revendications 1 à 9 en association avec un diluant ou support acceptable du point de vue pharmaceutique.

11. Utilisation d'un composé de formule (I) suivant l'une quelconque des revendications 1 à 9 ou d'un composé de formule

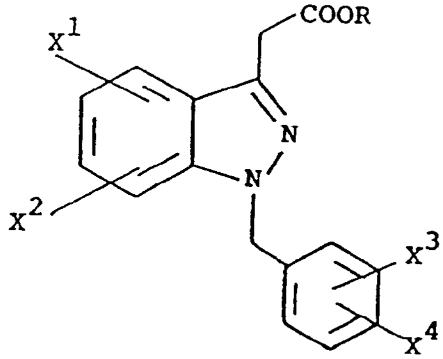

---(II)

dans laquelle R, $X^1$, $X^2$, $R^3$ et $X^4$ ont la définition donnée dans la revendication 1, pour la préparation d'un médicament destiné à combattre des complications diabétiques chez un mammifère atteint de diabète chronique.

**Revendications pour les Etats contractants suivants : ES, GR**

1. Procédé de production d'un composé de formule

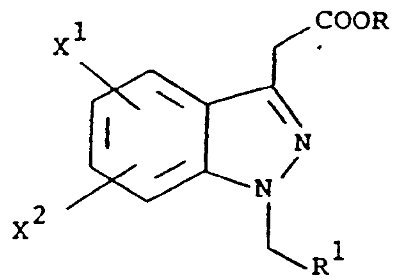

---(I)

dans laquelle

$X^1$ et $X^2$ représentent chacun, indépendamment, l'hydrogène, un radical fluoro, chloro, bromo, trifluorométhyle, alkyle en $C_1$ à $C_3$ ou alkoxy en $C_1$ à $C_3$ ;

R est l'hydrogène ou un radical formant un ester classique qui est hydrolysable dans des conditions physiologiques ;

$R^1$ représente

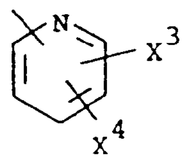 , 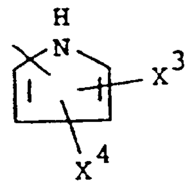 , 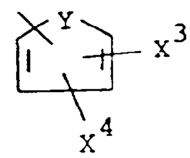 ,

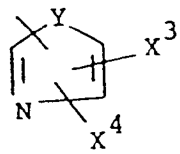 , 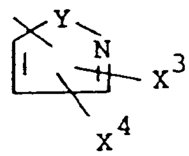 , 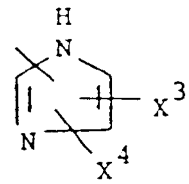 ,

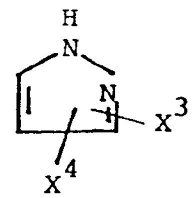 ou 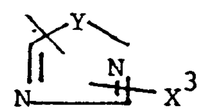 ;

Y représente le soufre ou l'oxygène ;

$X^3$, pris séparément, est l'hydrogène, un radical fluoro, chloro, bromo, trifluorométhyle, alkyle en $C_1$ à $C_3$, alkoxy en $C_1$ à $C_3$ ou

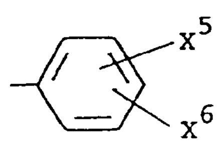 ;

$X^4$, pris séparément, $X^5$ et $X^6$ représentent chacun indépendamment l'hydrogène, un radical fluoro, chloro, bromo, trifluorométhyle, alkyle en $C_1$ à $C_3$ ou alkoxy en $C_1$ à $C_3$ ; et

$X^3$ et $X^4$, pris ensemble, forment conjointement avec les atomes adjacents de carbone auxquels ils sont attachés, un noyau benzénique portant les substituants $X^5$ et $X^6$ ;

d'un sel cationique pharmaceutiquement acceptable de ce composé lorsque R est l'hydrogène ; ou

d'un sel d'addition d'acide pharmaceutiquement acceptable de ce composé, qui comprend

(a) la réaction d'un composé de formule

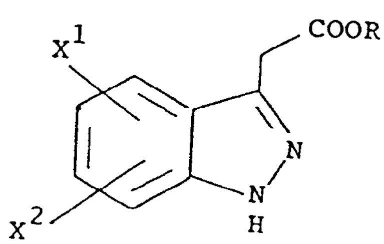 --- (IV)

avec un composé de formule

$$R^1\text{-}CH_2\text{-}X,$$

formules dans lesquelles R, $R^1$, $X^1$ et $X^2$ sont tels que définis ci-dessus et X est groupe partant susceptible d'un déplacement nucléophile, dans un solvant inerte vis-à-vis de la réaction en présence d'une base ; et la cas échéant

(b) lorsque R est l'hydrogène, la solvolyse classique d'un ester préformé de formule (I) dans laquelle R représente autre chose que l'hydrogène ;

(c) lorsque R représente autre chose que l'hydrogène, l'estérification classique d'un composé acide préformé de formule (I) dans laquelle R est l'hydrogène ;

(d) lorsque R est l'hydrogène, la transformation classique en un sel cationique acceptable du point de vue pharmaceutique ; ou

(e) la transformation classique en un sel d'addition d'acide.

2. Procédé suivant la revendication 1, dans lequel R représente un groupe :

1H-furanne-5-one-1-yle ;
1H-isobenzofuranne-3-one-1-yle ;
gamma-butyrolactone-4-yle ;
$-CH_2CH_2NR^2R^3$ ;
$-CHR^4OCOR^5$ ; ou
$-CHR^4OCOOR^6$ ;

où

$R^2$ et $R^3$, pris séparément, représentent chacun indépendamment un groupe alkyle en $C_1$ à $C_4$ ; ou forment conjointement avec l'atome d'azote auquel ils sont attachés un noyau de pyrrolidine, pipéridine ou morphorline ;

$R^4$ est l'hydrogène ou le groupe méthyle ;

$R^5$ est un groupe alkyle en $C_1$ à $C_6$, carboxy(alkyle en $C_1$ à $C_6$), carboxycyclohexyle ou carboxyphényle ; et

$R^6$ est un groupe alkyle en $C_1$ à $C_6$.

3. Procédé suivant la revendication 1, dans lequel R est l'hydrogène.

4. Procédé suivant la revendication 3, dans lequel $X^1$ est l'hydrogène ou le radical 5-chloro et $X^2$ est l'hydrogène.

5. Procédé suivant la revendication 4, dans lequel $R^1$ est un groupe

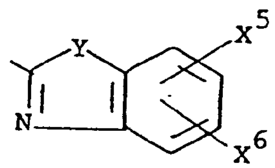

.

6. Procédé suivant la revendication 5, dans lequel Y est le soufre.

7. Procédé suivant la revendication 6, dans lequel $X^1$ est le radical 5-chloro, $X^5$ est l'hydrogène et $X^6$ est le radical 5-fluoro ou 5-trifluorométhyle.

8. Procédé suivant la revendication 6, dans lequel $X^1$, $X^5$ et $X^6$ représentent chacun un atome d'hydrogène.

9. Procédé suivant la revendication 6, dans lequel $X^5$ est le radical 5-fluoro et $X^6$ est le radical 7-fluoro.

10. Procédé de préparation d'une composition pharmaceutique destinée à combattre des complications diabétiques chroniques chez des mammifères, qui consiste à associer un composé de formule (I) ou un composé de formule

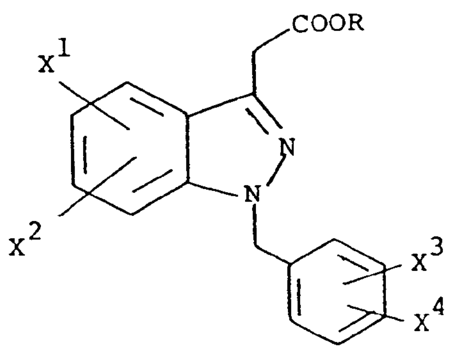

---(II)

dans laquelle R, $X^1$, $X^2$, $R^3$ et $X^4$ sont tels que définis dans la revendication 1,

un sel cationique pharmaceutiquement acceptable de ce composé lorsque R est l'hydrogène ; ou
un sel d'addition d'acide pharmaceutiquement acceptable de ce composé,
à un support acceptable du point de vue pharmaceutique.